# EUROPEAN PATENT SPECIFICATION

(11) **EP 3 210 578 B1**
(45) Date of publication and mention of the grant of the patent: **29.01.2020**
(21) Application number: 16203769.1
(22) Date of filing: 13.12.2016
(51) Int. Cl.: A61F 5/058, A61F 13/10, A61F 5/01

(54) **WRIST BRACE**
HANDGELENKBANDAGE
ORTHÈSE DE POIGNET

(30) Priority: 24.02.2016 IT UB20161033
(43) Date of publication of application: 30.08.2017
(73) Proprietor: Tenortho S.r.l. Unipersonale, 20853 Biassono (Monza Brianza) (IT)
(72) Inventor: TENTORIO, Alessio, 20052 MONZA (IT)
(74) Representative: Tansini, Elio Fabrizio

(56) References cited:
- WO-A1-01/08618
- US-A1- 2005 096 575
- US-A1- 2005 197 608
- US-A1- 2013 211 303

## Description

The present invention relates to a wrist elastic brace.

More specifically, the present invention relates to an elastic brace for the treatment of trauma, sprains, tendinitis, arthritis, fractures and fracture sequelae of the radius and ulna ends.

As is known, wrist elastic braces (commonly referred to as "wrist bands") have a flexible or semi-rigid structure, adapted to give a support capable of limiting the movement of the injured joint.

Generally, the wrist band is constituted by a main portion of elastic fabric wearable by the user so as to wrap the wrist, namely the zone defined between the terminal ends of ulna and radius and the palm/back of the hand.

The main portion is in the form of an open band so as to be wrapped and anchored to the wrist by means of adapted belts or Velcro® closures.

The main portion also has at least one through hole for accommodating the thumb. In this way, under these conditions of use (i.e., being wrapped on the wrist) the main portion defines both the hole for the thumb passage and an opening for the finger passage, adapted to make free the movement of the fingers themselves.

Generally, to allow both right and left-handed use of the above wrist band, specific major portions are configured to be wrapped on either the right wrist or the left wrist.

In this case, the hole for the thumb passage, the shape of the main portion and the respective closing bands of the brace, are appropriately positioned for a given application dedicated only to the left or the right wrist.

In addition, to implement a stabilization and containment action of the joint, the wrist bands are also equipped with rigid inserts (sticks) in the form of rods extending between the forearm and the dorsal and palmar side of the hand. These inserts are configured in view of the anatomy of the hand and wrist in order to give an ergonomic and maximal natural stabilization to the joint. For this purpose, for each brace, dedicated to a specific right- or left-handed use, the inserts have a predetermined shape for the specific use. However, the known braces described above has a drawback mainly due to the versatility in the use thereof for both right and left wrists. Consequently, in the case where the user has to operate in an alternating manner on both wrists, it is necessary to have two different braces, resulting in drawbacks in terms of practicality, costs and overall dimensions.

In this context, it should be considered that an early rheumatoid arthritis or osteoarthritis, which affects initially only one wrist, may progress to a disease that over time affects also the other wrist, resulting in a need to have two different braces.

In this case, therefore, the user initially needs a single brace for the left or right wrist, and then he/she must purchase a second elastic brace, with consequent drawbacks in terms of costs.

To overcome this drawback, elastic braces are provided to be used in an ambidextrous manner, therefore able to be dressed both around the right wrist and around the left wrist.

These elastic braces have a main portion having a shape almost symmetrical and provided with two holes for housing the thumb. The holes are adjacent to one another and each of which is positioned at a respective side of the main portion.

In this manner, the portion is adapted to both the right and the left arm, by the insertion of the thumb (either right or left) in the specific hole.

It is also known from document US 2013/211303 A1 a wrist brace having a flexible main body panel having first and second opposed sides, and first and second opposed surfaces having sections of hook receivable material. Even this type of brace, although capable of allowing an ambidextrous use with the consequent advantages in terms of cost and practicality, however, has a major drawback.

This drawback is due to the poor stabilization and containment action determined by the shape of the brace, which, in order to adapt to both wrists, does not wrap properly the single joint.

In this regard, note that, during the use, the presence of the two holes results in the formation of an exposed area of the joint resulted from the presence of the hole not occupied by the thumb.

This exposed (i.e., not wrapped) area extends at the hand and therefore allows the movement of the hand itself relative to the arm.

Moreover, to adapt the symmetrical areas of the main portion to the back and palm of the hand, internal semirigid inserts (sticks) are used able to adapt to the natural joint curvature.

Consequently, also the stabilization action given by the inserts appears to be inadequate to obtain a proper limitation of joint movements, making the brace unusable in the case of particular pathologies such as fractures of the ulna and radius ends.

In this context, the technical task underlying the present invention is to provide an elastic wrist brace, which overcomes the drawbacks of prior, art mentioned above.

In particular, it is an object of the present invention to provide an elastic brace that is versatile and thus usable for both the right and the left wrist. A further object of the present invention is to provide an ambidextrous elastic brace, which is structurally simple, inexpensive, comfortable and easy to wear by the user.

It is a further object of this invention to provide an elastic brace, which allows to intervene properly on the joint, by adapting to the configuration of the articulation regardless of the right- or left-handed use.

The technical task mentioned and the objects stated are substantially achieved by a wrist elastic brace, comprising the technical characteristics set out in one or more of the appended claims.

Further characteristics and advantages of the present invention will become more apparent from the description of an exemplary, but not exclusive, and therefore non-limiting preferred embodiment of a wrist elastic brace, as illustrated in the appended Figures, in which:
- Figure 1 shows a top plan view of the elastic brace according to the invention, in a respective condition of non-use, in which it is not applied on the wrist articulation;
Figure 2 shows a plan bottom view of the wrist elastic brace of Figure 1;
- Figures 3a and 3b are perspective views of the brace of Figure 1 in a condition of use, in which it is applied on a wrist, and in the respective views of the palmar and dorsal areas of the hand;
- Figures 4a, 4b and 4c show perspective views of respective construction details of the wrist elastic brace according to the present invention;
- Figures 5 and 5b show top plan views of a further constructive detail of the wrist elastic brace according to a second embodiment of the invention; and
- Figures 6a, 6b and 6c show perspective views of the elastic brace in the condition of use and according to the second embodiment, and in the respective views of the palmar, dorsal and side areas of the hand.

With reference to the accompanying Figures, 1 refers globally to a wrist elastic brace according to the present invention.

In particular, the elastic brace comprises a main body 2, made of flexible material and in the form of a band to be wrapped around the wrist articulation to be treated. As specified above, in the accompanying drawings a main body 2 coupled to a user's wrist is illustrated purely by way of example and without limitation.

Specifically, it should be noted that the main body 2 is arranged to be wrapped in correspondence of the forearm (end part of ulna and radius) and the hand. In this way, the brace 1 is configured to wrap both the dorsal and the palmar part of the hand, stabilizing the wrist by limiting its movement, while leaving the fingers free.

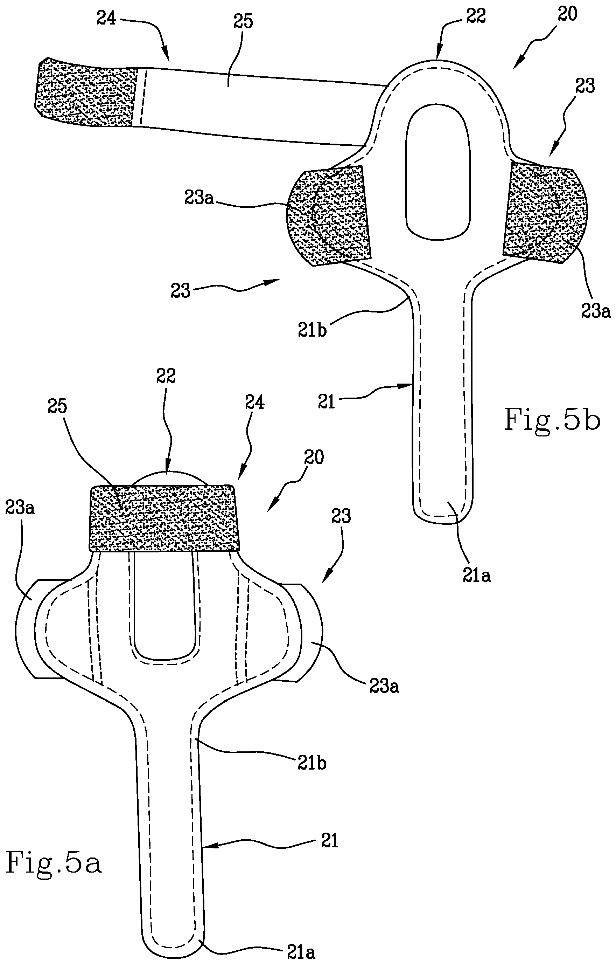

According to a second embodiment illustrated in Figures 5a to 6c, also the thumb is wrapped and stabilized for a more versatile use of the brace 1. In fact, in this configuration the brace 1 is also used in case of fracture of the thumb.

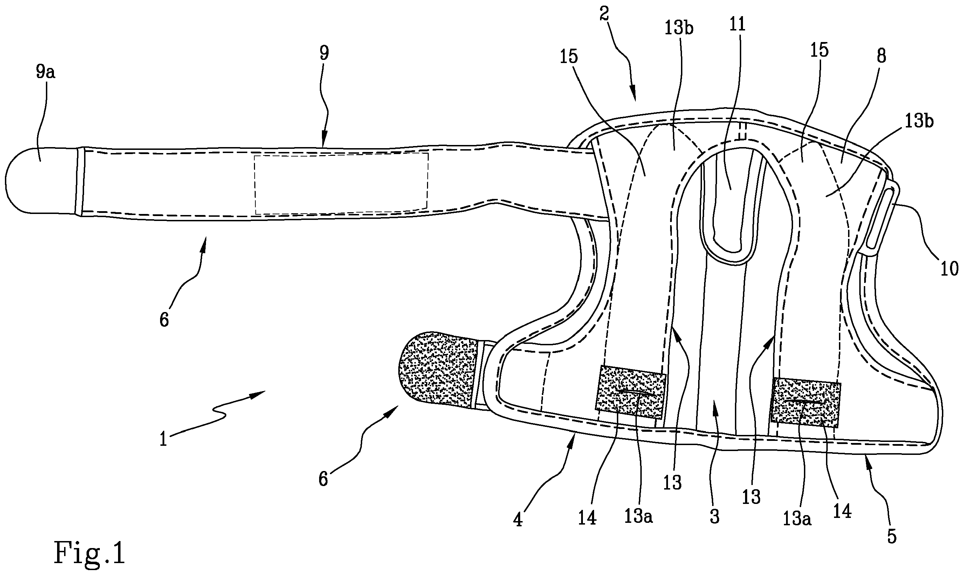

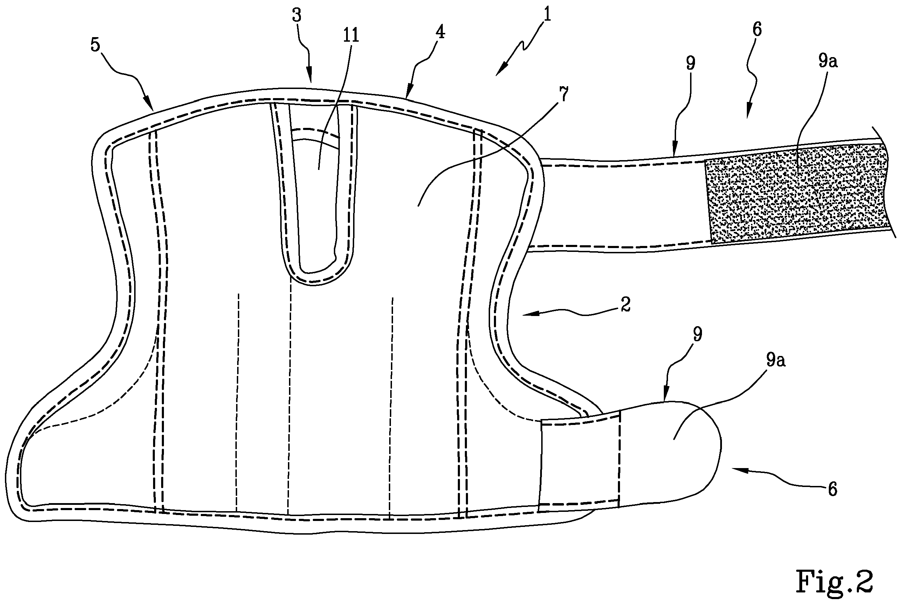

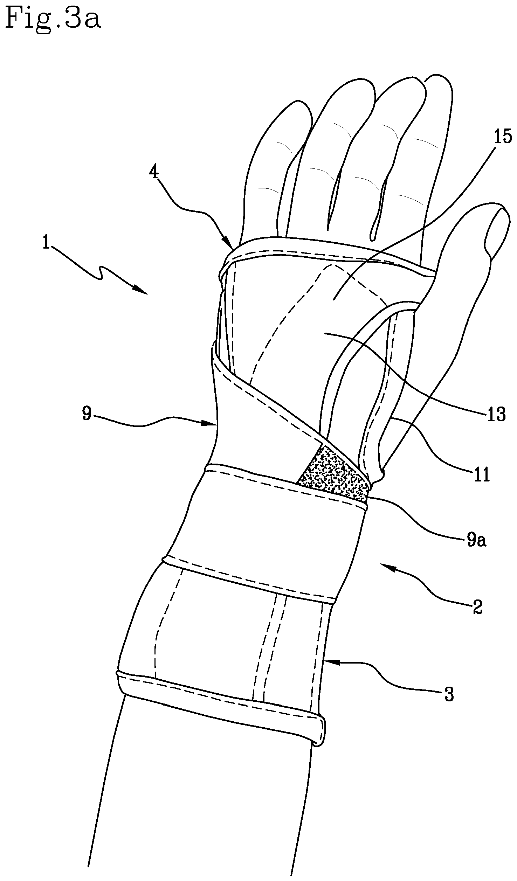

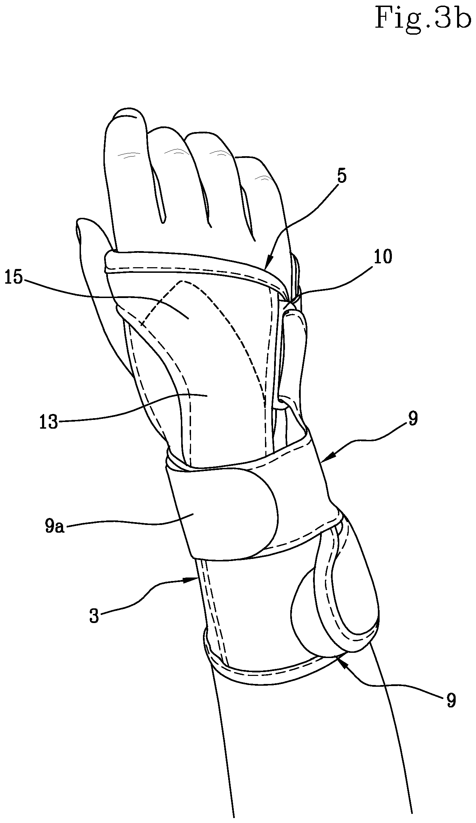

In greater detail, with reference to Figures 1 and 2, the main body 2 has a symmetrical conformation defining: a central portion 3 of the centre line, which, during the use, is arranged in correspondence of the thumb and the radius of the user (Figure 3a, and 3b); a first and a second side portion 4, 5 arranged, during the use, in correspondence of the dorsal or palmar side of the hand, respectively. Note that the two side portions 4, 5 are arranged on opposite sides of the central portion 3 and have a respective peripheral curvature adapted to conform to the profile of the wrist.

In addition, note that the main body comprises a hole 11 for housing the thumb, made at said central portion 3 of the centre line.

The brace 1 further comprises closing means 6 coupled to the main body 2 to engage in a stable manner the body 2 itself to the wrist articulation. Preferably, the closing means 6 engage, in a reversible manner, the first and the second portions 4, 5 with each other and around the wrist (Figures 3a, 3b).

The main body 2 also has an inner surface 7 that can be abutted to the articulation (Figure 2), and an outer surface 8 (Figure 1) opposite to the inner surface 7.

These surfaces are preferably made of elastic and breathable fabric, in order to allow the contact with the skin and to adapt to the size and to the development of the wrist.

In this context, note that the closing means 6 are constituted by at least one elastic band 9 projecting from the first side portion 4 and having one end 9a for anchoring to the outer surface 8 of the main body.

In greater detail, there are at least two elastic bands 9: a first band 9 next to the hand, which is inserted in a slot 10 formed in the second portion 5 (Figure 1 and 3b), to be folded and hooked on itself by means of appropriate Velcro surfaces; and a second band, distal from the hand, which overlaps to, and engages the outer surface 8 (Figure 2 and 3b) by means of appropriate Velcro surfaces.

The wrist elastic brace 1 comprises at least one rigid insert 12 associated to the main body 2 to stiffen at least partially the main body 2 itself and to limit the movement of the wrist articulation.

Advantageously, the rigid insert 12 is removably associable with the main body 2 in two operating configurations each of which corresponding to a respective use of the brace 1 for the right wrist or the left wrist.

In other words, in function of the rigid positioning of the insert 12, it is possible to use the main body 2 both to wrap a right wrist (as in Figures 3a and 3b) and a left wrist.

In particular, at least one of the side portions 4, 5 comprises an inner compartment 13 for housing the rigid insert 12. The compartment 13 has an access opening 14 (Figure 1) to connect/disconnect the insert 12 from the respective compartment 13, therefore defining the mentioned operating conditions of the brace 1 (for right- or left-handed use).

Note that the access opening 14 is formed at the outer surface 8 of the body 2 to allow access to the compartment 13 both in conditions of non-use and in conditions of use of the brace 1.

Preferably, the brace 1 comprises a plurality of inserts 12 that can be inserted in the respective compartments 13 defined in each side portion 4, 5.

In particular, with reference to Figure 1, each side portion 4, 5 has a compartment 13 for housing a respective insert 12.

The two compartments 13 are arranged at the sides of the central portion 3 and the hole 11, and each of which has a distal end 13a of the hole 11 for housing the thumb defining the respective access opening 14, and an end 13b proximal to the hole 11 defining a curvature 15 facing the hole 11 itself.

With reference to Figures 3a and 3b, the curvatures 15 of each compartment 13 extend, during the use, in correspondence of the back and palm of the hand.

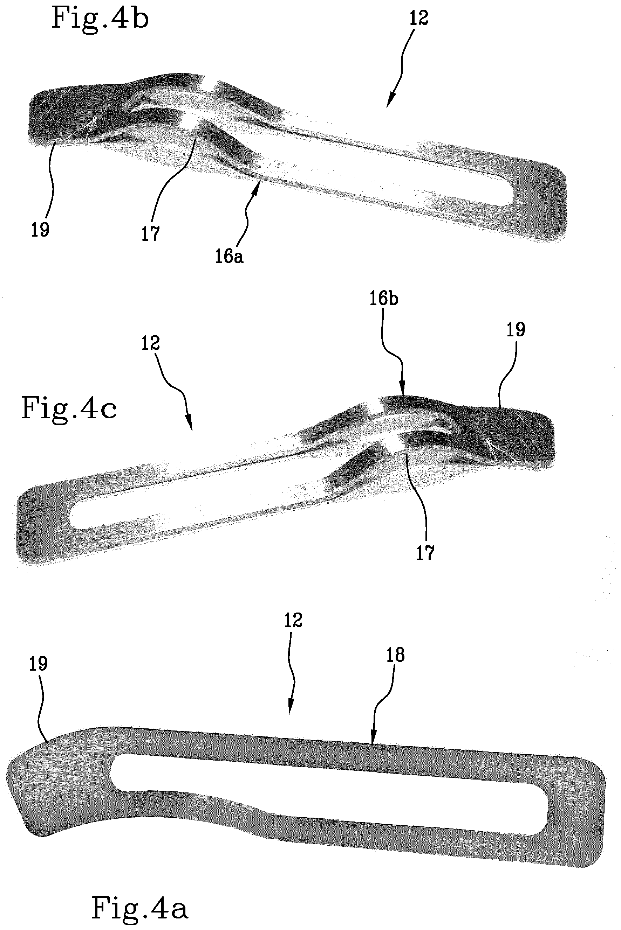

According to the Figures 4a 4c, the inserts 12 preferably comprise: at least a first and a second palmar insert 16a, 16b each of which consists of a flat rod, provided with a concave area 17 that can be located at the palm of the hand; and at least a dorsal insert 18 consists of a rod having a flat profile and arranged at the back of the hand.

Each palmar and dorsal insert 16a, 16b, 18 further has a curved end 19 facing the hole 11 for housing the thumb, arranged in correspondence of said curvature 15 of the compartment 13.

Consequently, the first palmar insert 16a (Figure 4b) is inserted into the compartment 13 of the second side portion 5 to wrap the body 2 on the left wrist.

The second palmar insert 16b (Figure 4c) is instead inserted into the compartment 13 of the first side portion 4 to wrap the body 2 on the right wrist.

In this configuration, the aforementioned concave area 17 defines, at the inner surface 7, a seat for housing the palm of the hand.

Advantageously, the palmar and dorsal inserts 16a, 16b, 18 are inserted into the respective compartment 13 of the first or second side portion 4, 5 to define a use at the left or the right wrist of the brace 1.

In other words, depending on the right or left use of the brace 1, the appropriate palmar insert 16a, 16b is inserted into one of the two compartments 13, through the access opening 14. Consequently, the dorsal insert 18 is inserted into the other compartment 13.

For example, with reference to Figures 3a and 3b, in which there is illustrated a use of the brace 1 for the right wrist, the second palmar insert 16b is inserted into the compartment 13 of the first side portion 4. In this situation, the dorsal insert 18 is inserted into the compartment 13 of the second side portion 5.

In case of use of the brace 1 for the left wrist, the second palmar insert 16b is replaced by the dorsal insert 18, which is then inserted into the compartment 13 of the first side portion 4.

Consequently, the first palmar insert 16a is inserted into the compartment 13 of the second side portion 5.

In this situation, note that the dorsal insert 18, having a flat configuration, is used for both right and left-handed use, turning it over by means of the respective generally curved end areas 19 according to the curvature 15 of the compartment 13.

Advantageously, through the replacement of the inserts 12, the brace 1 can be used for both right and left wrists, while maintaining a single hole for housing the thumb.

The inserts 12, preferably made of rigid material such as aluminium or other technically equivalent material, are further internally hollowed in order to give greater lightness to the material itself.

Still, according to the second embodiment illustrated in Figures 5a to 6c, the brace 1 further comprises an anatomical interaction element 20 associable to the main body 2 at the hole 11 to limit the movement of the thumb.

The anatomical interaction element 20 is applied in the event of fracture of the thumb, in order to give a dual stabilization to the joint of the wrist and to the thumb.

As is illustrated in Figures 5a and 5b, the interaction element 20 is made of a rigid material and has: a rod 21 provided with a first end 21a insertable within the hole 11 for housing the thumb; and an annular portion 22 disposed at a second end 21b of the rod 21 opposite the first end 21a. The annular portion 22 is arranged around the dorsal area of the thumb to engage the thumb itself to the main body 2.

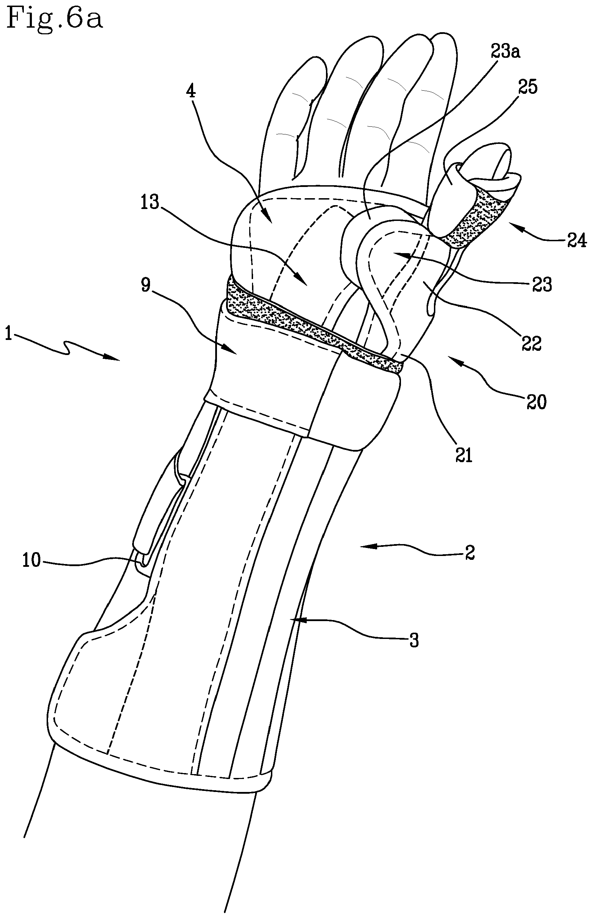

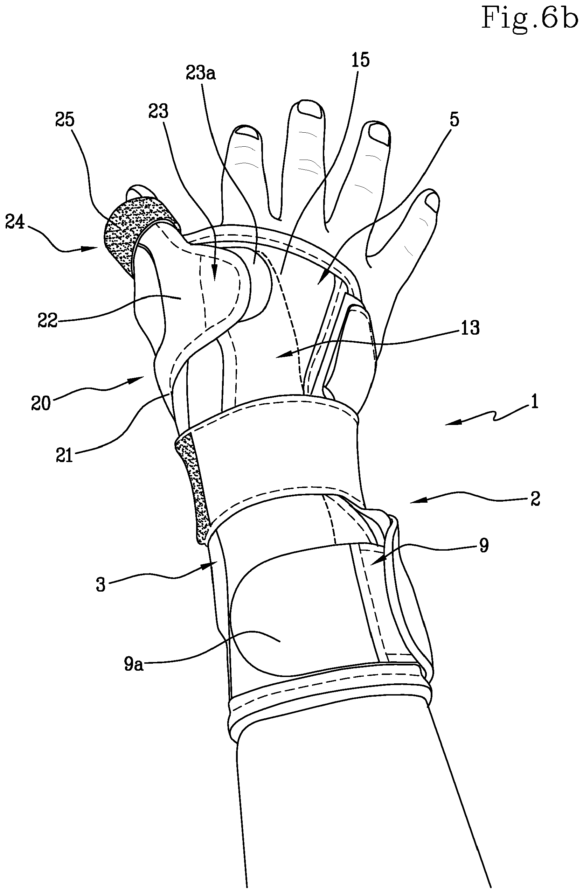

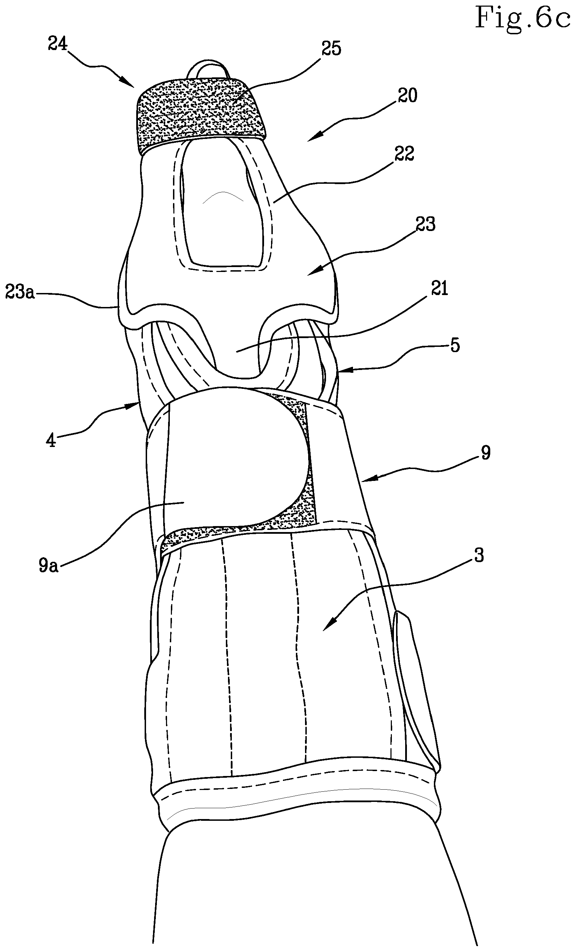

Note that the annular portion 22 defines an opening within which the metacarpal of the thumb is comprised (Figures 6a, 6b, 6c).

The element 20 is also made of a not elastic (to limiting the movement of the thumb) and breathable fabric to provide greater comfort of the element itself during the use.

The annular portion 22 also has first anchoring means 23 projecting from opposite sides of the annular portion 22 and arranged to engage on the outer surface 8 of the main body 2.

Note in particular that the first anchoring means 23 consist of Velcro bands 23a engageable at the two side portions 4, 5 around the hole 11.

Second means 24 for anchoring the thumb are also provided, to firmly hold the thumb itself. The second anchoring means 24 are constituted by a Velcro band 25, which is wrapped around the thumb and anchored thereon.

As is illustrated in the views of Figures 6a, 6b and 6c, the band 25 retains the thumb at the respective distal phalanx, to stabilize it on the element 20.

Advantageously, the coupling of the element 20 to the body 2 is performed simply by inserting the rod 21 into the hole 11 and engaging it between the radius and the central portion 3. Instead, by means of the first anchoring means 23 the annular portion is engaged to the body 2.

Consequently, the element 20 is easily applicable, regardless of the of the right- or left-handed use of the body 2, to the thumb, for a greater versatility of the brace 1, which can be used both for the stabilization of the wrist only and for the combined treatment of wrist and thumb.

Furthermore, the elastic brace 1 is particularly versatile because it can be adapted for both right and left wrists, while maintaining the correct operation characteristics in the stabilization action given by rigid inserts 12. The brace 1 is furthermore structurally simple and inexpensive, as able to be used for both wrists, and it is easy to wear. In this regard, note that the user is able to wear the brace 1 autonomously, by previously introducing the appropriate inserts 12 in their respective compartments 13 depending on the right- or left-handed use.

## Claims

1. Elastic brace comprising:
- a main body (2), made of elastic material that can be wound at least partially around a respective wrist articulation;
- a plurality of rigid inserts (12) associated to the main body (2) to stiffen at least partially the main body (2) itself and to limit the movement of the wrist articulation;
- closing means (6) coupled to said main body (2) to engage in a stable manner said main body to the wrist articulation;
said rigid inserts (12) being removably associable with the main body (2) in two operating configurations each of which corresponding to a respective use of the brace (1) for the right wrist or the left wrist;
**characterized in that** said inserts (12) comprise:
- at least a first and a second palmar insert (16a, 16b) each of which consists of a flat rod, provided with a concave area (17) that can be located at the palm of the hand, each palmar insert (16a, 16b) having a generally curved end area (19) facing the hole (11) for housing the thumb; and
- at least a dorsal insert (18) consists of a rod having a flat profile and arranged at the back of the hand, said dorsal insert (18) having a generally curved end area (19) facing the hole (11) for housing the thumb.

2. Brace according to the previous claim, **characterized in that** said main body has a symmetrical shape defining:
- a central portion (3) of the centre line arranged in use at the thumb and the radius bone;
- a first side portion (4) arranged in use at the palmar or dorsal side of the hand;
- a second side portion (5) arranged in use at the palmar or dorsal side of the hand;
said closing means (6) engaging, in a reversible manner, the first and the second portions (4, 5) with each other and around the wrist.

3. Brace according to the previous claim, **characterized in that** said main body (2) comprises a hole (11) for housing the thumb and made at said central portion (3) of the centre line.

4. Brace according to claim 2 or 3, **characterized in that** at least one of said side portions (4, 5) comprises an inner compartment (13) for housing said rigid insert (12); said compartment (13) having an access opening (14) to connect/disconnect the insert (12) from said compartment (13) defining the respective operating conditions.

5. Brace according to the previous claim, **characterized in that** said main body (2) has an inner surface (7) that can be abutted to the articulation, and an outer surface (8) opposite to the inner surface (7); said access opening (14) extending at said outer surface (8).

6. Brace according to any one of claims 2 to 5, **characterized in that** it comprises a plurality of inserts (12) and **in that** each side portion (4, 5) comprises a respective inner housing compartment (13) for housing one of said inserts (12); said compartments (13) being arranged at the sides of the hole (11) for housing the thumb.

7. Brace according to claim 1, **characterized in that** each compartment (13) has a distal end (13a) from the hole (11) for housing the thumb defining said access opening (14) of the insert (12), and a proximal end (13b) to the hole (11) for housing the thumb defining a curvature (15) facing said hole (11) for the containment of the generally curved end area (19) of the respective rod; said palmar (16a, 16b) and dorsal inserts (18) being inserted in the respective compartment (13) of the first or second side portions (4, 5) to define a use at the left or right wrist of the brace (1).

8. Brace according to any one of claims 2 to 7, **characterized in that** said closing means (6) comprise at least one elastic band (9) projecting from the first side portion (4) and having one end (9a) for anchoring to the outer surface (8) of the main body (2); said first portion (4) being at least partially superimposable on the second portion (5).

9. Brace according to any one of claims 2 to 8, **characterized in that** it further comprises an anatomical interaction element (20) associable to said main body (2) at the hole (11) to limit the movement of the thumb.

10. Brace according to the previous claim, **characterized in that** said interaction element (20) is made of a rigid material and has:
a rod (21) provided with a first end (21a) insertable within the hole (11) for housing the thumb;
and an annular portion (22) arranged at a second end (21b) of the rod opposite the first (21a); said annular portion (22) extending around the thumb.

11. Brace according to the previous claim, **characterized in that** said annular portion (22) further comprises first means (23) for anchoring to the outer surface (8) of the main body (2), and second means (24) for anchoring to the thumb to stably retaining the thumb itself to the main body (2).

## Patentansprüche

1. Elastische Bandage umfassend:
- einen Hauptkörper (2), hergestellt aus elastischem Material, der mindestens teilweise um ein jeweiliges Handgelenk gewickelt werden kann;
- eine Vielzahl an starren Einsätzen (12), die mit dem Hauptkörper (2) assoziiert sind, um den Hauptkörper (2) selbst mindestens teilweise zu versteifen und um die Bewegung des Handgelenks zu begrenzen;
- Schließmittel (6), die mit dem Hauptkörper (2) gekoppelt sind, um den Hauptkörper stabil mit dem Handgelenk einzugreifen;
wobei die starren Einsätze (12) entfernbar mit dem Hauptkörper (2) in zwei Betriebskonfigurationen assoziierbar sind, von denen jede einer jeweiligen Verwendung der Bandage (1) für das rechte Handgelenk oder das linke Handgelenk entspricht; **dadurch gekennzeichnet, dass** die Einsätze (12) umfassen:
- mindestens einen ersten und einen zweiten palmaren Einsatz (16a, 16b), von denen jeder aus einer flachen Stange besteht, die mit einem konkaven Bereich (17) versehen ist und die auf der Handfläche platziert werden kann, wobei jeder palmare Einsatz (16a, 16b) einen allgemein gekrümmten Endbereich (19) aufweist, der dem Loch (11) zur Aufnahme des Daumens zugewandt ist; und
- mindestens ein dorsaler Einsatz (18) besteht aus einer Stange aufweisend ein flaches Profil und ist am Handrücken angeordnet, wobei der dorsale Einsatz (18) einen allgemein gekrümmten Endbereich (19) aufweist, der dem Loch (11) zur Aufnahme des Daumens zugewandt ist.

2. Bandage nach dem vorhergehenden Anspruch, **dadurch gekennzeichnet, dass** der Hauptkörper eine symmetrische Form aufweist, definierend:
- einen zentralen Abschnitt (3) der Mittellinie, der in Verwendung am Daumen und am Radiusknochen angeordnet ist;
- einen ersten Seitenabschnitt (4), der in Verwendung an der palmaren oder dorsalen Seite der Hand angeordnet ist;
- einen zweiten Seitenabschnitt (5), der in Verwendung an der palmaren oder dorsalen Seite der Hand angeordnet ist;
wobei die Schließmittel (6) den ersten und den zweiten Abschnitt (4, 5) miteinander und um das Handgelenk reversibel eingreifen.

3. Bandage nach dem vorhergehenden Anspruch, **dadurch gekennzeichnet, dass** der Hauptkörper (2) ein Loch (11) zur Aufnahme des Daumens umfasst und an dem zentralen Abschnitt (3) der Mittellinie bereitgestellt ist.

4. Bandage nach Anspruch 2 oder 3, **dadurch gekennzeichnet, dass** mindestens einer der Seitenabschnitte (4, 5) eine innere Unterteilung (13) zur Aufnahme des starren Einsatzes (12) umfasst; wobei die Unterteilung (13) eine Zugangsöffnung (14) zum Verbinden/Trennen des Einsatzes (12) mit/von der Unterteilung (13) aufweist, die die jeweiligen Betriebsbedingungen definiert.

5. Bandage nach dem vorhergehenden Anspruch, **dadurch gekennzeichnet, dass** der Hauptkörper (2) eine Innenfläche (7), die an das Gelenk anschlagen kann, und eine der Innenfläche (7) gegenüberliegende Außenfläche (8) aufweist; wobei sich die Zugangsöffnung (14) an der Außenfläche (8) erstreckt.

6. Bandage nach einem der Ansprüche 2 bis 5, **dadurch gekennzeichnet, dass** sie eine Vielzahl an Einsätzen (12) umfasst und dadurch, dass jeder Seitenabschnitt (4, 5) eine jeweilige innere Gehäuseunterteilung (13) zur Aufnahme eines der Einsätze (12) umfasst; wobei die Unterteilungen (13) an den Seiten des Lochs (11) zur Aufnahme des Daumens angeordnet sind.

7. Bandage nach Anspruch 1, **dadurch gekennzeichnet, dass** jede Unterteilung (13) ein vom Loch (11) distales Ende (13a) zur Aufnahme des Daumens, das die Zugangsöffnung (14) des Einsatzes (12) definiert, und ein zum Loch (11) proximales Ende (13b) zur Aufnahme des Daumens aufweist, das einer dem Loch (11) zugewandten Krümmung (15) zum Enthalten des allgemein gekrümmten Endbereichs (19) der jeweiligen Stange definiert;
wobei die palmaren (16a, 16b) und die dorsalen Einsätze (18) in die jeweilige Unterteilung (13) des ersten oder zweiten Seitenabschnitts (4, 5) eingesetzt sind, um eine Verwendung am linken oder rechten Handgelenk der Bandage (1) zu definieren.

8. Bandage nach einem der Ansprüche 2 bis 7, **dadurch gekennzeichnet, dass** die Schließmittel (6) mindestens ein elastisches Band (9) umfassen, das von dem ersten Seitenabschnitt (4) vorsteht und ein Ende (9a) zur Verankerung an der Außenfläche (8) des Hauptkörpers (2) aufweist; wobei der erste Abschnitt (4) dem zweiten Abschnitt (5) mindestens teilweise überlagert werden kann.

9. Bandage nach einem der Ansprüche 2 bis 8, **dadurch gekennzeichnet, dass** sie zudem ein anatomisches Interaktionselement (20) umfasst, das an dem Loch (11) mit dem Hauptkörper (2) assoziierbar kann, um die Bewegung des Daumens zu begrenzen.

10. Bandage nach dem vorhergehenden Anspruch, **dadurch gekennzeichnet, dass** das Interaktionselement (20) aus einem starren Material hergestellt ist und aufweist:
eine Stange (21), die mit einem ersten Ende (21a) versehen ist, das in das Loch (11) zur Aufnahme des Daumens einsetzbar ist;
und einen ringförmigen Abschnitt (22), der an einem zweiten Ende (21b) der Stange gegenüber der ersten (21a) angeordnet ist; wobei sich der ringförmige Abschnitt (22) um den Daumen erstreckt.

11. Bandage nach dem vorhergehenden Anspruch, **dadurch gekennzeichnet, dass** der ringförmige Abschnitt (22) zudem erste Mittel (23) zur Verankerung an der Außenfläche (8) des Hauptkörpers (2) und zweite Mittel (24) zur Verankerung an dem Daumen umfasst, um den Daumen selbst am Hauptkörper (2) stabil zu halten.

## Revendications

1. Orthèse élastique comprenant :
- un corps principal (2) constitué d'un matériau élastique pouvant être enroulé au moins partiellement autour d'une articulation du poignet respective ;
- une pluralité d'inserts rigides (12) associés au corps principal (2) pour raidir au moins partiellement le corps principal (2) lui-même et pour limiter le mouvement de l'articulation du poignet ;
- des moyens de fermeture (6) couplés au dit corps principal (2) pour mettre en prise de façon stable ledit corps principal sur l'articulation du poignet ; lesdits inserts rigides (12) pouvant être associés de façon amovible au corps principal (2) dans deux configurations de fonctionnement chacune d'elle correspondant à une utilisation respective de l'orthèse (1) pour le poignet droit ou le poignet gauche ;
**caractérisée en ce que** lesdits inserts (12) comprennent :
- au moins un premier et un second insert palmaire (16a, 16b) chacun d'eux consistant en une tige plate pourvue d'une zone concave (17) pouvant être située en correspondance de la paume de la main, chaque insert palmaire (16a, 16b) comportant une zone d'extrémité (19) généralement incurvée faisant face à l'orifice (11) pour loger le pouce ; et
- au moins un insert dorsal (18) consistant en une tige comportant un profil plat et disposé au dos de la main, ledit insert dorsal (18) comportant une zone d'extrémité (19) généralement incurvée faisant face à l'orifice (11) pour loger le pouce.

2. Orthèse selon la revendication précédente, **caractérisée en ce que** ledit corps principal a une forme symétrique définissant :
- une partie centrale (3) de la ligne centrale disposée, en fonctionnement, en correspondance du pouce et du radius ;
- une première partie latérale (4) disposée, en fonctionnement, en correspondance du côté palmaire ou dorsal de la main ;
- une seconde partie latérale (5) disposée, en fonctionnement, en correspondance du côté palmaire ou dorsal de la main ;
lesdits moyens de fermeture (6) se mettant en prise, de façon réversible, avec les première et seconde parties (4, 5) réciproquement et autour du poignet.

3. Orthèse selon la revendication précédente, **caractérisée en ce que** ledit corps principal (2) comprend un orifice (11) pour loger le pouce et réalisé en correspondance de ladite partie centrale (3) de la ligne centrale.

4. Orthèse selon la revendication 2 ou 3, **caractérisée en ce qu'**au moins une des parties latérales (4, 5) comprend un compartiment interne (13) pour loger ledit insert rigide (12) ; ledit compartiment (13) comportant une ouverture d'accès (14) pour relier/décrocher l'insert (12) dudit compartiment (13) définissant les conditions de fonctionnement respectives.

5. Orthèse selon la revendication précédente, **caractérisée en ce que** ledit corps principal (2) comporte une surface intérieure (7) pouvant se mettre en butée contre l'articulation, et une surface extérieure (8) opposée à la surface intérieure (7) ; ladite ouverture d'accès (14) se prolongeant en correspondance de ladite surface extérieure (8).

6. Orthèse selon l'une quelconque des revendications de 2 à 5, **caractérisée en ce qu'**elle comprend une pluralité d'inserts (12) et **en ce que** chaque partie latérale (4, 5) comprend un compartiment de logement (13) interne respectif pour loger un desdits inserts (12) ; lesdits compartiments (13) étant disposés sur les côtés de l'orifice (11) pour loger le pouce.

7. Orthèse selon la revendication 1, **caractérisée en ce que** chaque compartiment (13) comporte une extrémité distale (13a), à partir de l'orifice (11) pour loger le pouce, définissant ladite ouverture d'accès (14) de l'insert (12), et une extrémité proximale (13b), sur l'orifice (11) pour loger le pouce, définissant une courbure (15) faisant face au dit orifice (11) pour contenir la zone d'extrémité (19) généralement incurvée de la tige respective ;
lesdits inserts palmaires (16a, 16b) et dorsaux (18) étant introduits dans le compartiment respectif (13) des première ou seconde parties latérales (4, 5) pour définir une utilisation au poignet gauche ou droit de l'orthèse (1).

8. Orthèse selon l'une quelconque des revendications de 2 à 7, **caractérisée en ce que** lesdits moyens de fermeture (6) comprennent au moins une bande élastique (9) dépassant de la première partie latérale (4) et comportant une extrémité (9a) pour s'ancrer à la surface extérieure (8) du corps principal (2) ; ladite première partie (4) étant au moins partiellement superposable à la seconde partie (5).

9. Orthèse selon l'une quelconque des revendications de 2 à 8, **caractérisée en ce qu'**elle comprend de plus un élément d'interaction (20) anatomique associable au dit corps principal (2) en correspondance de l'orifice (11) pour limiter le mouvement du pouce.

10. Orthèse selon la revendication précédente, **caractérisée en ce que** ledit élément d'interaction (20) est constitué d'un matériau rigide et comporte :
une tige (21) pourvue d'une première extrémité (21a) pouvant s'introduire à l'intérieur de l'orifice (11) pour loger le pouce ;
et une partie annulaire (22) disposée en correspondance d'une seconde extrémité (21b) de la tige opposée à la première (21a) ; ladite partie annulaire (22) se prolongeant autour du pouce.

11. Orthèse selon la revendication précédente, **caractérisée en ce que** ladite partie annulaire (22) comprend de plus des premiers moyens (23) pour s'ancrer à la surface extérieure (8) du corps principal (2), et des seconds moyens (24) pour s'ancrer au pouce pour retenir stablement le pouce lui-même au corps principal (2) .
